# EUROPEAN PATENT SPECIFICATION

(11) **EP 3 448 305 B1**
(45) Date of publication and mention of the grant of the patent: **30.12.2020**
(21) Application number: 17715051.3
(22) Date of filing: 09.03.2017
(51) Int. Cl.: A61C 19/05, A61C 19/04, A61F 5/56, A61B 5/107

(54) **INSTRUMENT FOR MEASURING THE POSITION OF THE LOWER JAW AND METHOD WHEREBY SUCH AN INSTRUMENT IS APPLIED AND PRODUCED**
INSTRUMENT FOR MEASURING THE POSITON OF THE LOWER JAW AND METHOD WHEREBY SUCH AN INSTRUMENT IS APPLIED AND PRODUCED
INSTRUMENT DE MESURE DE LA POSITION DE MÂCHOIRE INFÉRIEURE, ET MÉTHODE PAR LAQUELLE UN TEL INSTRUMENT EST APPLIQUÉ ET PRODUIT

(43) Date of publication of application: 06.03.2019
(73) Proprietor: Novoden Tec BVBA, 2000 Antwerpen (BE)
(72) Inventor: BRAEM, David, 2000 Antwerpen (BE)
(74) Representative: Schneiders & Behrendt PartmbB Rechtsanwälte - Patentanwälte
(86) International application number: PCT/EP2017/055569
(87) International publication number: WO 2018/162068

(56) References cited:
- WO-A1-03/092562
- WO-A1-2006/040380
- WO-A2-2010/141868

## Description

The present invention relates to an instrument for measuring the position of the lower jaw.

Instruments for measuring the position of the lower jaw are known, e.g., from WO 2010 / 141868 A2 and WO 03 / 092562 A1.

More specifically, the invention is intended for measuring the position of the lower jaw with respect to the upper jaw with awake, anaesthetised and/or unconscious patients in order to assess and establish the effect of the forward positioning of the lower jaw on the air passage in the upper airway.

It is known that with certain patients the upper airway collapses at one or more levels while sleeping. These patients can suffer from snoring and/or 'obstructive sleep apnoea' (OSA) or the obstructive sleep apnoea syndrome (OSAS).

It is now generally accepted that the moving forward or protrusion of the lower jaw or mandible can improve the stability in this upper airway.

In general, this invention relates to an instrument that accurately measures the individual dynamic positioning of the lower jaw, with awake patients, and with anaesthetised and/or unconscious patients, for the purpose of examining and determining the most optimum forward position of the lower jaw prior to the treatment of snoring and/or OSA and/or OSAS. This invention can equally be used for examining of a patient on an intermediary basis during treatment in order to verify and/or adjust treatment of snoring and/or OSA and/or OSAS.

Changes in the accessibility of the upper airway are in general simulated by the forced and/or guided moving forward or protrusion of the lower jaw or mandible with awake and/or anaesthetised and/or unconscious patients. The effects of such a determined protrusion on the air passage in the upper airway are then evaluated making use of e.g. nasendoscopic techniques in a sedated or awake patient. Such a determined protrusion of the lower jaw is often tried out in patients with obstructive sleep apnoea who qualify for the use of a therapy with an oral correction device for which a prior accurate measurement of the desired lower jaw protrusion is useful.

The attending physician faces a number of problems when trying to evaluate the effects of the mandibular protrusion on the air passage in the upper airway by means of the so-called "chin lift" manoeuvre:
1. The forced protrusion, in general known as the "chin lift" manoeuvre, results in a mandibular position that is almost irreproducible, which impedes the accurate measurement as well as the determination of the desired protrusion;
2. This forced protrusion is moreover an extreme manoeuvre and it is improbable that a patient would tolerate this position of the lower jaw resulting from this, and is thus of minimal clinical value for patients who qualify for the use of an oral correction device;
3. Further, the forced protrusion has an intrinsically variable and uncontrolled vertical component whereby the mouth is undesirably opened, depending on how the manoeuvre is performed, which again is difficult to reproduce;

The attending physician faces a number of problems when trying to evaluate the effects of the mandibular protrusion on the air passage in the upper airway by means of known measuring devices:
1. Once the optimum protrusive position has been selected by means of an instrument for measuring the position of the lower jaw, there are no means to accurately and dynamically convey this position, whereby ideally, together with the limit positions of maximum protrusion and retrusion, the protrusive path between these extreme positions must also be converted into the oral correction device that must be produced by the dental technician.
2. Furthermore, the appreciation of the midline position of the lower jaw during the registration and following procedures is not possible with known instruments.
3. Still further, the handling of known instruments is difficult, especially for left-handed physicians. A suited instrument for both, left- and right-handed physicians is not available.

Despite its difficulties and inadequacies, the "chin lift" manoeuvre described above remains the most generally used method for determining the air passage through the upper airway, irrespective of its incapacity to convey the diagnosed optimum horizontal and vertical relationship between the upper and lower jaw as it exists in the individual patient.

The reason for this is that up to now nobody has developed a reproducible measurement method, further referred to as the bite registration, that can easily perform a correct position measurement of the lower jaw of the patient and can then convey this position, together with the protrusive path, to the therapeutic oral correction devices such that the bite registration also enables the protrusive path to be checked during the production of the correction device.

There are a number of measuring instruments that are able to accurately make bite registrations, but they lack the dynamic component of the back and forth guidance of the lower jaw and they are difficult to handle and an appreciation of the midline position of the lower jaw during the registration is not possible.

Such a measuring instrument is the George meter (U.S. Pat. No. 5.154.069 of Peter George, dated 13 October 1992). Once the bite registration has been made, no further adaptation and/or check of the protrusive path can be made as the bite registration determines the lower jaw in a fixed position with respect to the upper jaw and so does not allow further movement of the lower jaw. Furthermore, such instruments can only be used on awake patients as they rely on voluntarily mandibular movements.

Another measuring instrument that enables the horizontal and vertical position of the lower jaw to be measured in the patient and which enables the individual positioning of the teeth has been developed by SomnoMed. Its capacity to register the retrusion and protrusion positions as well as the vertical positions using a stepwise wedge at the incisor region, while a precise positioning of the sagittal movement is made, makes the device precise, accurate and reproducible. However, once a position has been chosen and the bite registration has been made, no further adaptation and/or check of the protrusive path is possible as the bite registration determines the lower jaw in a fixed position with respect to the upper jaw and so does not allow further movement of the lower jaw.

Another measuring instrument that can determine the position of the lower jaw with respect to the upper jaw or maxilla in three dimensions, including the vertical, is the Andrea Gauge (US 2004/0241464 A1). Its capacity to register the retrusion and protrusion positions and the vertical positions, while a precise positioning of the sagittal movement is made, makes the device precise, accurate and reproducible. Again, once a position has been chosen and the bite registration has been made, no further adaptation and/or check of the protrusive path is possible as the bite registration determines the lower jaw in a fixed position with respect to the upper jaw and so does not allow further movement of the lower jaw.

Another measuring instrument that enables the horizontal and vertical position of the lower jaw to be measured in the patient and which enables the individual positioning of the teeth has been developed by Holstrom (U.S. Pat. No. 7.832.403 2B of Holstrom et al., dd. 16 November 2010). This measuring instrument has the disadvantage that the parts that are placed over the teeth of the upper jaw and the lower jaw, and which take up space in the mouth, are very voluminous and due to their volume push the tongue away, such that changes in the accessibility of the upper airway are introduced that no longer relate to the snoring and/or apnoeic events or which even cause displacements of the surrounding soft tissue that counteract the possible favourable effects of the mandibular protrusion.

There is currently no method known in the art that enables the operator of the measuring instrument:
(1) to register easily the optimum protrusion for transfer to an oral correction device that has to be produced, including the capacity for an in vitro check of the protrusive path.
(2) to individually adapt the measuring instrument to the patient with a minimal disturbing effect on the surrounding soft tissues in the mouth;
(3) to easily and dynamically position the lower jaw of the patient accurately and reproducibly both before, during and after the bite registration;

WO 2006 / 040380 A1 discloses a bite ring with an eccentrically arranged handle.

The object of the present invention is to provide an improved instrument for the position measurement of the lower jaw as defined in the claims.

According to the invention, an instrument is disclosed which is provided with a first and a second bite fork that are individually adaptable to the upper and lower tooth arch of a patient, whereby the first and the second bite fork are each attached to an extension that extends in a horizontal plane, so that the extensions extend outside the mouth of the patient in front of the anterior teeth when the first and the second bite fork are adapted to the upper and lower tooth arch, and whereby the extensions enable the bite forks to move with respect to one another in a horizontal movement direction restricting lateral deviation of the lower jaw, while the instrument is placed in the patient's mouth and enables the registration of the relative position of the bite forks and thus the relative position of the upper and lower tooth arch while extending outside the patient's mouth, wherein the first and the second bite fork have the same symmetric shape with regard to a midline position of the first and the second bite fork.

The instrument of the invention enables easy dynamical positioning of the lower jaw of the patient including the appreciation of the midline position during measurement in a minimal invasive way, whereby the movement of the first bite fork with respect to the second bite fork proceeds steplessly. The symmetric shape with regard to a midline position of the first and the second bite fork makes the handling of the instrument easier, especially for left- handed as well as right-handed physicians. Due to the identical symmetric shape of the first and the second bite fork with regard to a midline position of the first and the second bite fork, left-handed and right-handed physicians can easily change the orientation by turning the instrument a half rotation around the horizontal direction of the movement.

According to the invention, the instrument of the invention is provided with extensions that are arranged eccentrically to a midline position of the first and the second bite fork. Eccentric positioning of the extensions allows the appreciation of the midline position of the lower jaw during registration and following procedures due to an unobstructed view on the interincisive midline both in the upper and in the lower jaw. Moreover the eccentric positioning of the extensions avoids interference of the bite registration with, e.g., a vertical post of an articulator so that the extensions need not to be shortened during further procedures and allows the invention to be used irrespective of the type of articulator preferred by the technician involved in the fabrication of an oral correction device.

In a preferred embodiment of the instrument, a first thermoplastic layer is releasably attachable on the first bite fork and a second thermoplastic layer is releasably attachable on the second bite fork. In this fashion, the basis of the instrument being the first and the second bite fork can be re-used for different patients after proper sterilisation. When using the instrument, impressions of the teeth of the upper and lower jaws are generated in the respective thermoplastic layer. In an alternative embodiment of the instrument of the invention, the first thermoplastic layer is fixedly arranged on the first bite fork and the second thermoplastic layer is fixedly arranged on the second bite fork. In this disposable embodiment, the instrument is immediately usable, but cannot be re-used for another patient. In an alternative embodiment of the instrument of the invention, both thermoplastic layers can be replaced with any appropriate curing moulding compound at any of the following stages of the procedure. Such a compound is releasably or fixed attachable on the first bite fork and on the second bite fork. When using the instrument, impressions of the teeth of the upper and lower jaws are generated in the respective curing moulding compound layer.

In a further preferred embodiment of the invention, the thermoplastic layers can be made malleable by warming up. By using thermoplastic materials that can be made malleable, for example by submersing in warm water, the instrument can be used very efficiently without the need to prepare an appropriate curing moulding compound and to apply it to the bite forks in a number of successive processing steps.

According to a further preferred embodiment of the invention, the first and the second bite fork have projections or retention holes to fix the thermoplastic layers on the first and the second bite fork or to fix the appropriate curing moulding compound. The mutual movement between the first and second bite fork remains possible because the material used for the bite registration does not impede the opposite fork. For this purpose, the retention holes for the registration material do not reach through the fork but have a thin seal at the underside. This prevents the material used for the bite registration getting into contact with the opposite fork. The retention holes are optimised for use with a low-temperature thermoplastic overmoulding. They provide retention, so that the low-temperature thermoplastic material cannot be pulled out of the holes during bite-registration or during endoscopy later on. This can be achieved by providing undercuts in the retention holes on the bite forks. The same geometry of the retention holes provides retention when using appropriate curing moulding compound.

In yet another preferred embodiment of the invention, a protrusion adjustment screw is arranged between the extensions of the first and the second bite fork allowing the physician to control the movement of the extensions with respect to one another in the horizontal movement direction. The protrusion is realised and adjusted by rotating the adjustment screw regardless of the voluntary movements of the lower jaw by the patient. This pull-screw, preferably made of material that is not interfering with radiographical imaging techniques, acts like a worm-mechanism, so it is self-locking and it keeps its position, which is ideal for use in endoscopy and said medical imaging scanners. The adjustment screw is useful for very fine adjustments, or to apply more power, e.g. during endoscopy or in a scanner. The total adjustable range of the adjustment screw is preferably 30mm: from -10mm (lower teeth backward) to +20mm (lower teeth forward).

In a further preferred embodiment, the protrusion adjustment screw is arranged at the end of the extensions of the first and the second bite fork with a rotational axis in the horizontal direction of the movement. This makes the protrusion adjustment screw easily accessible for left-handed and right-handed physicians. In a further preferred embodiment, the protrusion adjustment screw can easily be unlocked to allow again free movement of the lower jaw.

According to another preferred embodiment of the invention, a locking screw fixes the first and second bite fork when the physician desires to do so, preventing that the first and the second bite fork do slide freely with respect to one another in the horizontal movement direction. With the locking screw therefore a preferred position of the first bite fork and the second bite fork relative to each other can be fixed. The instrument can be taken out of the mouth of the patient in a position fixed with the locking screw, so that the relative position of the first and second bite fork remains preserved. This fixed position can be used for producing an oral correction device appropriate for the patient.

In a preferred embodiment, the locking screw is arranged on the right side or left side of the extensions with a rotational axis in the horizontal plane locking the movement of the extensions with respect to one another. In this way, the screw is easy to reach and it provides a high clamping force, without bending the device. The locking screw is positioned sideways for easy accessibility for left-handed and right-handed physicians. The screw can easily be relocated for left-handed or right-handed use by turning the instrument a half rotation around the horizontal direction. Optionally, two or more set screws could be provided on the left or / and the right side, if a higher clamping force is required.

According to a further preferred embodiment of the invention, the vertical height of the first and the second bite fork with regard to the horizontal plane can be increased by click-on steps. Hence, the present invention also enables the thickness of the first and/or second bite fork to be adapted to the patients' characteristics and thus the vertical component of the registration to be kept variable. This can be done by, but is not limited to, click-on steps fastened in the existing retention holes, but also by adapting the thickness of the bite forks. An advantage of this adaptation in the vertical dimension is that it enables the effect of the opening of the lower jaw during the forward movement of the lower jaw to be examined. This is possible in a reversible fashion as the changes in the vertical dimension after and/or during the examination are adjustable as a function of the respective findings. An advantage of this design is that each desired position in the protrusive path as well as the entire protrusive path itself can be conveyed afterwards to any oral correction device that must be produced using known materials for teeth impressions. The click-on steps are provided for example, but not limited to, with a thickening, in the form of end pieces for the first bite fork and/or for the second bite fork. Such a click-on step is generally a plate-shaped element with protrusions that fit in the retention holes of the first or the second bite fork and which is provided with different thicknesses in steps of, e.g., 1.0 mm. Such a click-on step enables the thickness of the first and/or second bite fork to be adjusted to the needs of the examination and/or the characteristics of the patient.

According to still a further preferred embodiment of the invention, the extensions of the first and/or the second bite fork are detachable so that the extensions can be attached to other bite forks. This makes the instrument flexible for the use with different shapes and sizes of bite forks adapted to the physiology of the patient.

Both the first and the second bite fork may advantageously taper dorsally with regard to the backside of the mouth, following the natural convex shape curve of the tooth arcs. The advantage of this design is that the amount of material, especially the thickness of the bite forks, introduced in between both tooth arcs is minimal and does not add to unwanted increase of the interincisal distance once conveyed afterwards to any oral correction device that must be produced.

In a further preferred embodiment, provision is made for an integrated sliding part over the extensions, which not only serves to hold the extensions together during use, but also acts as means for the operator to make the desired markings on the top extension that correspond to the measured positions of the lower jaw, i.e. at least the maximum protrusion and retrusion, and the protrusion position chosen for insertion of an oral correction device that has to be produced on the basis of this chosen protrusion position. An advantage of such a sliding part is that it enables the instrument for measuring the position of the lower jaw to be used in clinical conditions whereby the patient can be awake or unconscious, and can be used as an aid to measure the optimum relative position of the first and second bite forks for the purpose of optimum accessibility of the upper airway.

After removal of the individual bite forks with extensions from the mouth of the patient, they can be used to convey the position of the lower jaw with respect to the upper jaw to a simulator, e.g. an articulator, by means of the present invention.

In a preferred embodiment, the individual bite forks with extensions, after removal from the mouth of the patient, can be placed back in the mouth at later times if necessary or desired, in an accurate position in the mouth of the same patient to enable monitoring of individual patients over time. An additional advantage of these accurate replaceable bite forks is that they thus enable the changes in the relative position of the upper and lower jaw to be quantitatively and accurately monitored over time, in order to supervise the patients and monitor any tooth movements as a function of time.

Preferably, the components of the instrument of the invention are made of materials suitable for automated production methods such as injection moulding of polymers, numerically controlled milling machines for metals, or robotics or other automated production methods. In very specific cases, the components are made of sterilisable materials.

The invention also relates to a method for the use of the above-described instrument, the method comprising at least the following steps:
1) Warming up a first and a second thermoplastic layer to make them malleable or, alternatively, put sufficient amounts of curing moulding compound;
2) Introduction of the first bite fork against the upper tooth arch by putting the first thermoplastic layer or curing moulding compound on the upper tooth arch of the patient whereby the inter-incisor line between the two upper central frontal incisors is in line with a midline position of the first and the second bite fork and whereby the outermost vestibular plane of these upper frontal incisors is positioned against a first edge with a first notch, again with the inter-incisor line between both frontal incisors in line with the first notch;
3) Introduction of the second bite fork against the lower tooth arch by putting the second thermoplastic layer or curing moulding compound on the lower tooth arch of the patient whereby the inter-incisor line between the two lower central frontal incisors is in line with a midline position of the first and the second bite fork and whereby the outermost vestibular plane of these lower frontal incisors is positioned against a second edge with a second notch, again with the inter-incisor line between both frontal incisors in line with the second notch;
4) Letting the thermoplastic layers or curing moulding compound harden until the upper and lower frontal incisors are positioned on the first and the second bite fork respectively;
5) Loosening of the set screw that fixes the first and the second bite fork so that the first and the second bite fork can slide with respect to one another in a horizontal movement direction, whereby the movement of the extensions with respect to one another in the horizontal movement direction is controlled with the protrusion adjustment screw when active, or freely when the protrusion adjustment screw is detached;
6) Gradual and gentle forward movement of the patient's lower jaw from a backward position to a preferred position in order to then register this position for the patient between the front and back position on the instrument by marking ;
7) Screwing in the set screw on the instrument so that the bite forks no longer move with respect to one another;
8) Taking the instrument out of the mouth and using the instrument as an aid for the production of an oral correction device for the patient.

This method is advantageous since it is not particularly unpleasant for the patient and is quickly completed. In this way, the optimal position for the lower jaw can be determined very quickly and easily and in a controllable and adjustable way since both forks are still able to move one against the other during and after the registration of the bite.

The invention also relates to a method for the production of an instrument, the method comprising the following steps:
1) Provision of a first and a second bite fork;
2) Inserting the first and the second bite fork to a mould and closing the mould;
3) Injection of an overmould in the mould to produce a thermoplastic layer on the first and the second bite fork;
4) Setting of the overmould in the mould;
5) Ejection of the first and the second bite fork from the mould.

This method is particularly advantageous because the instrument with the overmould can be manufactured very quickly and easily.

A preferred embodiment of the above production method, the first and the second bite fork are preheated before the injection of the overmould in the mould. Preheating the forks before the overmould makes the production more reliable, because both parts have preferably an equal temperature and do shrink more equally when cooling down.

With the intention of better showing the characteristics of the invention, a preferred embodiment of the instrument for measuring the position of the lower jaw according to the invention is described hereinafter by way of an example, without any limiting nature, with reference to the accompanying drawings, wherein:
- Figure 1: schematically shows a perspective view of a first bite fork of an instrument for measuring the position of the lower jaw according to the invention;
- Figure 2: schematically shows a perspective view of a second bite fork of an instrument for measuring the position of the lower jaw according to the invention;
- Figure 3: shows the natural habitual positioning with the tooth arches of a set of teeth almost against one another;
- Figure 4: shows the first bite fork of the instrument according to figure 1 and the second bite fork of the instrument according to figure 2 assembled;
- Figure 5: shows the instrument according to figure 4 with a detailed view on the extensions and the screws;
- Figure 6: shows a perspective view of a cross-section of the extensions and the set screw;
- Figure 7: shows the instrument according to figure 4 but with thermoplastic layers on the first bite fork and the second bite fork;
- Figure 8: shows a different perspective of the assembled instrument according to figure 4.

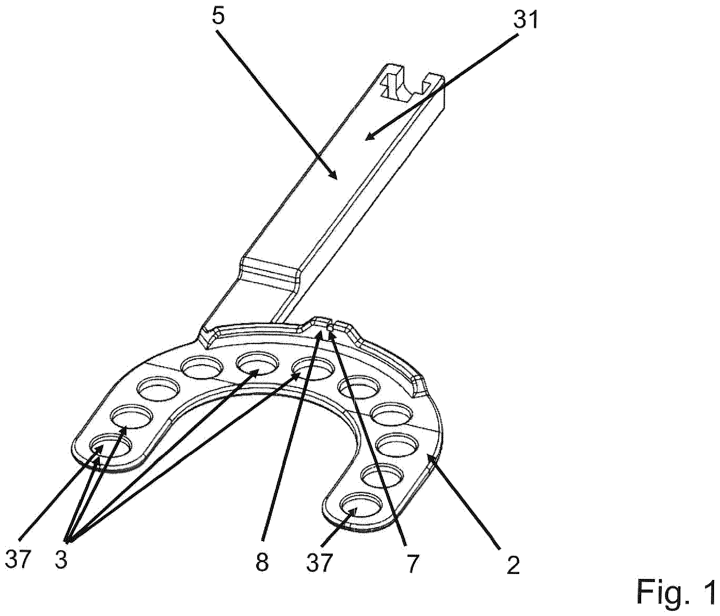

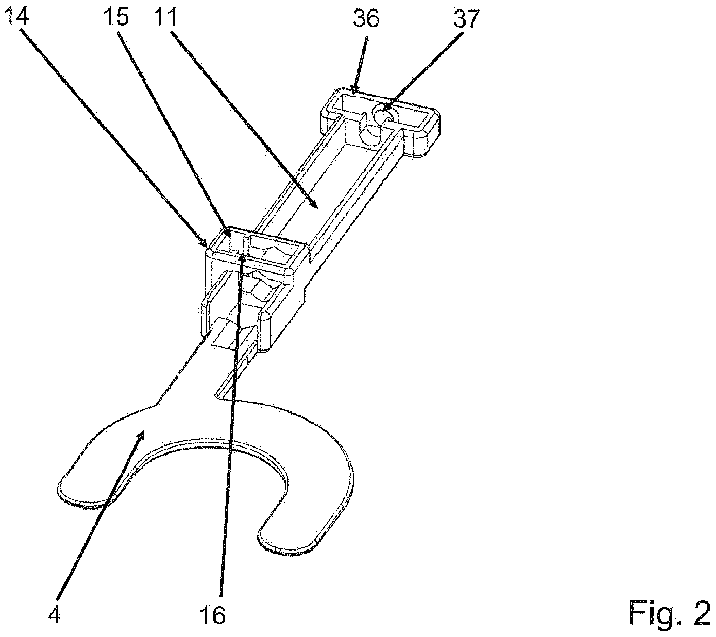

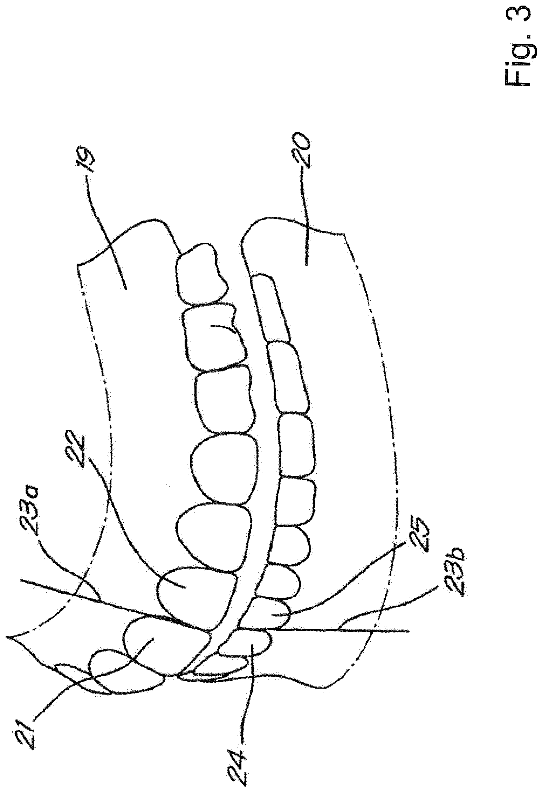

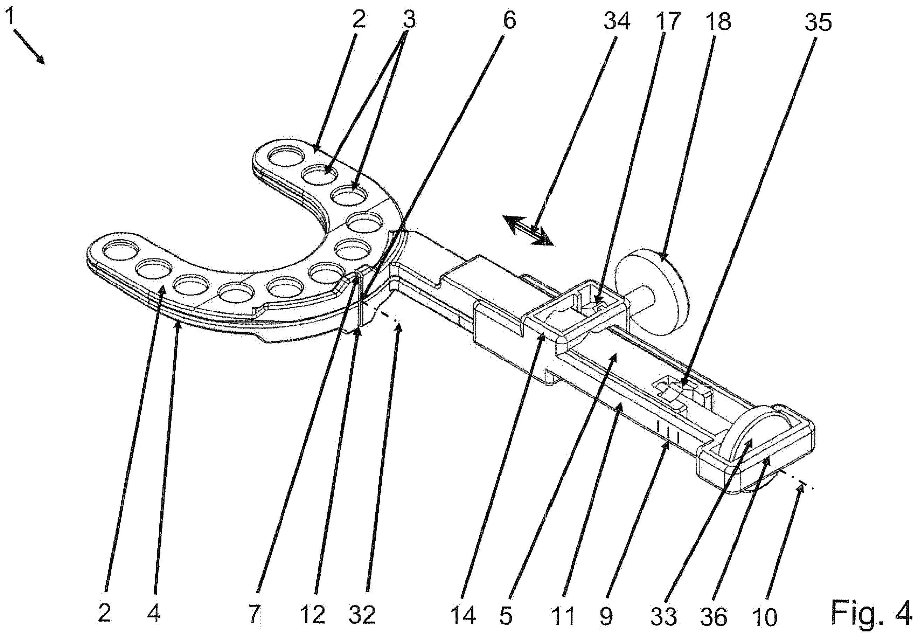

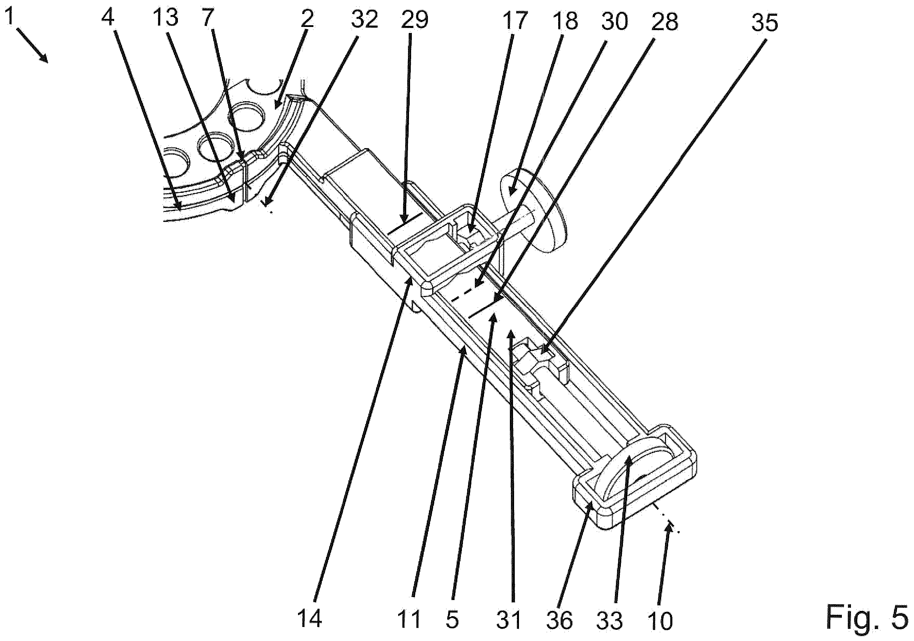

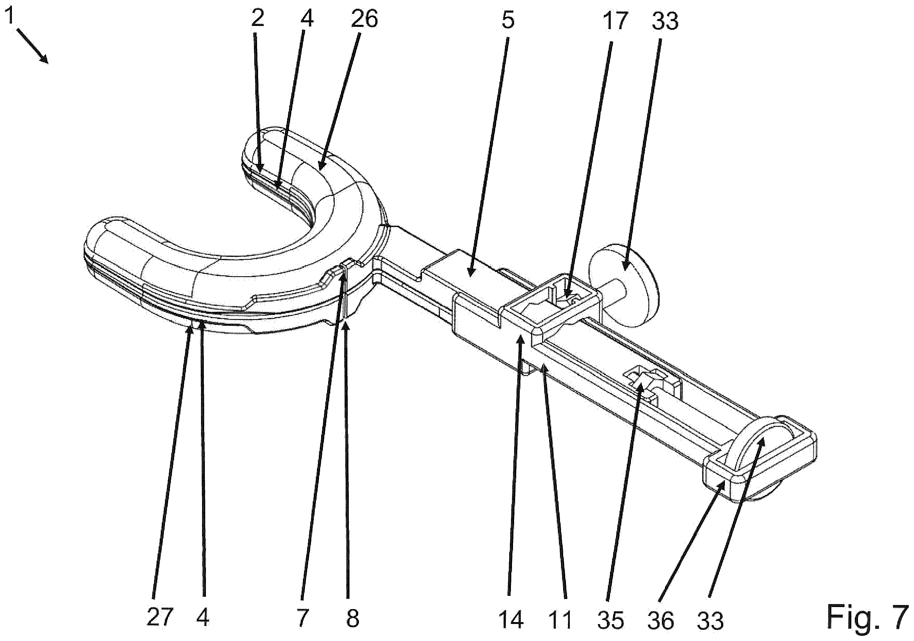

Figure 1 shows a perspective view of the first bite fork 2 of an instrument 1 (figure 5) for measuring the position of the lower jaw according to the invention, with retention holes 3 for the mechanical anchoring of the thermoplastic layer 26, 27 (figure 7) or the curing moulding compound, to be applied later. The retention holes 3 are sealed at the bottom by a thin seal 37 over the entire surface of the bite forks 2, 4 (figure 2) so that the material of the thermoplastic layer 26, 27 (figure 7) from the one bite fork 2 is not able to penetrate to the other bite fork 4. The first bite fork 2 is provided with an extension 5, whose longitudinal axis 10 (figure 4) is arranged eccentrically to the midline position 32 (figure 4), also marked by the notch 7. This notch 7 of an upright edge 8 is positioned against the upper 21, 22 (figure 3) or lower 24, 25 (figure 3) frontal incisors of the patient in the mouth. In the absence of these teeth the alignment of the invention can be carried out according to the midline of the facial structures, instead of the interincisive line 23a, 23b (figure 3).

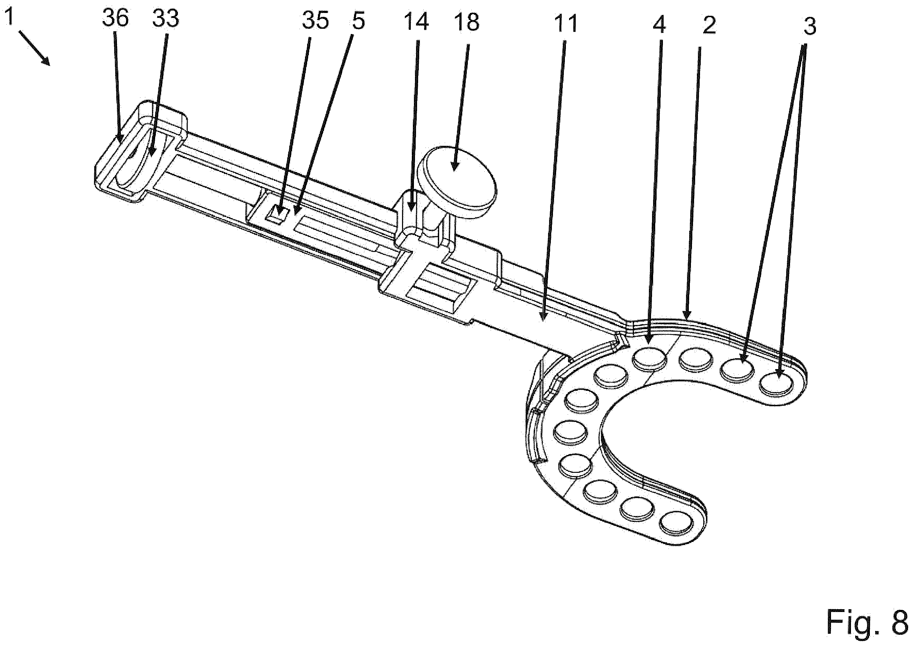

Figure 2 shows a perspective view of the second bite fork 4 of an instrument 1 for measuring the position of the lower jaw according to the invention. The sealed retention holes 3 for the mechanical anchoring of the thermoplastic layer 26, 27 (figure 7) to be applied later are on the backside of the fork as shown in figure 8. The second bite fork 2 is provided with an extension 11. The longitudinal axis 10 (figure 4) of the extension 11 of the second bite fork 4 is arranged eccentrically to the midline position 32 (figure 4), also marked by the notch 12 (figure 4) of a downward edge 13 (figure 5). This notch 12 is positioned against the upper 21, 22 (figure 3) or lower 24, 25 (figure 3) frontal incisors of the patient in the mouth. The second bite fork 4 is provided with an extension 11 that leads to a sliding part 14 that is provided with a window 15, through which the first extension 5 (figure 1) can be seen when it is introduced (figure 4). The sliding part 14 is provided with a cutaway 16 for a screw bolt 17 (figure 4) that engages with a set screw 18 (figure 8) that goes through it up to against the extension 5 (figure 1, 4) of the first bite fork 2 (figure 1, 4). The second bite fork 4 has an end part 36 with a hole 37 for the protrusion adjustment screw 33 (figure 4).

Figure 3 shows the natural habitual position of a set of teeth with the upper tooth arch 19 and the lower tooth arch 20 at some distance from one another, in which the natural position of the upper frontal incisors 21, 22 separated by the inter-incisor line 23a are shown, with respect to the lower frontal incisors 24, 25 separated by their inter-incisor line 23b.

Figure 4 shows the first bite fork 2 of the instrument 1 according to figure 1 and the second bite fork 4 of the instrument 1 according to figure 2 assembled together and adjusted by the protrusion adjustment screw 33 and fixed by the set screw 18. The first 2 and the second 4 bite fork are individually adaptable to the upper 19 (figure 3) and lower 20 (figure 3) tooth arch of a patient. The extension 5, 11 of the first 2 and second 4 bite fork extend in a horizontal plane, so that the extensions 5, 11 extend outside the mouth of the patient in front of the anterior teeth 21, 22, 24, 25 (figure 3) when the first 2 and the second 4 bite fork are adapted to the upper 19 (figure 3) and lower 20 (figure 3) tooth arch, and whereby the extensions 5, 11 enable the bite forks 2, 4 to move with respect to one another in a horizontal movement direction 34, while the instrument 1 is placed in the patient's mouth and enables the relative position of the bite forks 2, 4 and thus the relative position of the upper and lower tooth arch 19, 20 (figure 3) to be registered outside the patient's mouth. Moreover Figure 4 shows that the first 2 and the second 4 bite fork have the same symmetric shape with regard to a midline position 32, also marked by the notches 7, 12, of the first 2 and the second 4 bite fork. The protrusion adjustment screw 33 is arranged in an end part 36 of the second extension 11 between the extensions 5, 11 of the first 2 and the second 4 bite fork controlling the horizontal movement of the extensions 5, 11 with respect to one another in the horizontal movement direction 34. The protrusion adjustment screw 33 is easily accessible for left-handed and right-handed physicians, because it is arranged at the end of the extensions 5, 11 of the first 2 and the second 4 bite fork with a rotational axis in the horizontal direction of the movement 34. After the adjustment by the protrusion adjustment screw 33 the set screw 18 fixes the first 2 and second 4 bite fork in the desired position so that the first 2 and the second 4 bite fork do not slide freely with respect to one another in the horizontal movement direction 34. The set screw 18 is easily accessible for left-handed and right-handed physicians, because it is arranged on the right side or left side of the extensions 5, 11. Due to the same symmetric shape of the first 2 and the second 4 bite fork with regard to a midline position 32 of the first 2 and the second 4 bite fork left-handed and right-handed physicians can easily change the side of the set screw 18 by turning the instrument 1 a half rotation around the horizontal direction of the movement 34. The sliding part 14 not only keeps the extensions of the first 2 and the second 4 bite fork together, it supports also the operator of the instrument 1 to make the desired markings 28, 29, 30 (figure 5) on the extension 5, 11, corresponding to a certain number of positions of the lower jaw, i.e. at least the maximum protrusion 28 (figure 5) and retrusion 29 (figure 5), and the selected protrusion position 30 (figure 5) for the production of an oral correction device. The instrument 1 can be placed back at later times if necessary or desired after removal from the mouth of the patient, in an accurate position in the mouth of the same patient to enable monitoring of individual patients over time. The first 2 and the second 4 bite fork taper dorsally with regard to the backside of the mouth, following the natural convex shape curve of the tooth arcs 19, 20 (figure 3).

Figure 5 shows in more detail the sliding part 14 to which the extension 11 of the second bite fork 4 leads and in which a set screw 18 can be screwed against the extension 5 of the first bite fork 2 that slides through the sliding part 14 so that the second bite fork 4 is fixed with respect to the first bite fork 2 and no longer moves. The instrument 1 is placed in the mouth of the patient without fixation of the set screw 18 for measuring the position of the lower jaw, and positioned by his frontal incisors 21, 22, 24, 25 (figure 5). Figure 5 also shows the end of the extensions 5, 11 with the protrusion adjustment screw 33 in more detail. The protrusion adjustment screw 33 is arranged with a rotational axis in the horizontal direction of the movement 34 allowing adjustments concerning the protrusion between the first 2 and the second 4 bite fork by simply rotating the protrusion adjustment screw 33. The physician can also select a free sliding mechanism by removing the protrusion adjustment screw 33 at the extension 11. This can be done easily by unscrewing until the protrusion adjustment screw 33 comes loose from the second screw bolt 35 and then clicking the screw upwards out of the extension 11. Thereafter the first bite fork 2 slides freely in the second bite fork 4. When using the instrument 1 in the free-sliding mode, the position can still be locked using the set screw 18. Figure 5 also shows the top of the extension 5 of the first bite fork 2, on which three markings are made, i.e. the maximum horizontal movement of the second bite fork 4 forwards 28, backwards 29, and the chosen intermediate position 30, which will be used for marking the oral correction device and in this case is the only one marked by a dashed line to distinguish it from the most forward and most backward positions. The flat top surface of the extensions 5, 11 can be used to write markings on, e.g. with a permanent alcohol marker. Also on the side walls there is some room for writing, e.g. the patient's name, initials or identification number. For best adhesion of the marker ink, the surfaces to write on preferably have a satin finish. A metric mm-scale 9 (figure 4) could also be printed on the top face or side faces of the extensions 5, 11. The bite registration and measured protrusions can then be transferred to a dental laboratory for production of the corresponding correction device.

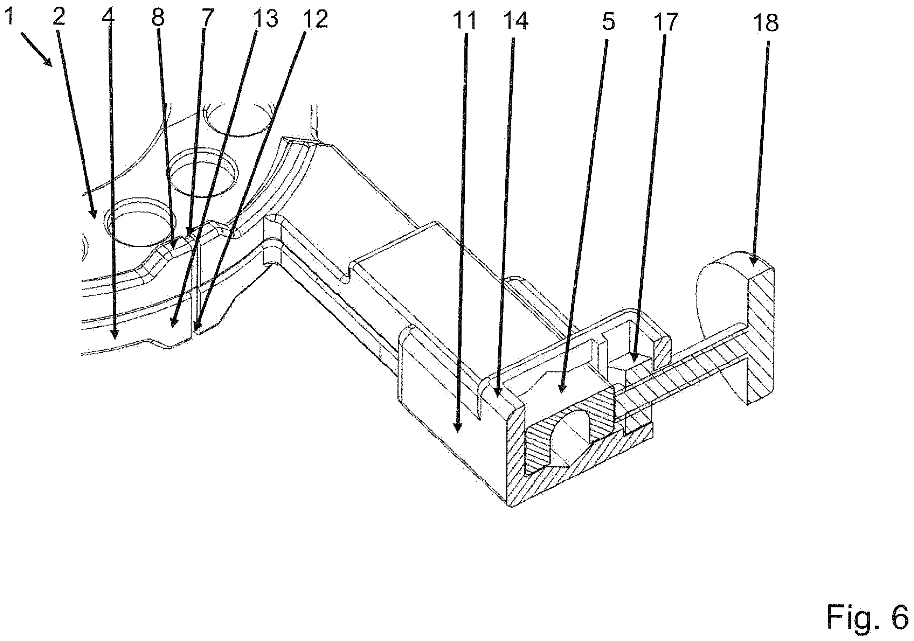

Figure 6 shows a cross-section in the rotational axis of the set screw 18. The sliding part 14 of the extension 11 of the second bite fork 4 guides the extension 5 of the first bite fork 2. The forks 2, 4 can be fixed with the set screw 18 by screwing it against the extension 5 of the first bite fork 2. A screw bolt 17 arranged in the sliding part 14 engages with the set screw 18 that goes through it against the extension 5 of the first bite fork 2.

Figure 7 shows the instrument 1 with thermoplastic layers 26, 27 on the first bite fork 2 and the second bite fork 4. A first thermoplastic layer 26 is arranged on the first bite fork 2 and a second thermoplastic layer 27 is arranged on the second bite fork 4. Another embodiment is that the first thermoplastic layer 26 is releasably attachable on the first bite fork 2 and the second thermoplastic layer 27 is releasably attachable on the second bite fork 4. The thermoplastic layers 26, 27 are fixed in the retention holes 3 and can be made malleable by warming up.

Figure 8 is a perspective view showing the instrument 1 from the bottom. The protrusion adjustment screw 33 and the set screw 18 are also easily accessible from this side. So left-handed and right-handed physicians can use the instrument 1 by turning it a half rotation around.

The components 2, 3, 4, 5, 11, 14, 18 of the instrument 1 are made of materials suitable for automated production methods such as injection moulding of polymers, numerically controlled milling machines for metals, or robotics or other automated production methods, including those for sterilisable materials.

The area of application of the instrument 1 for measuring the position of the lower jaw enables a dynamic bite registration to be measured in a patient who:
a) still has his own teeth, including crowns and/or bridges or otherwise placed over his own teeth or placed directly or indirectly on implants;
b) or has a partial removable prosthesis;
c) or has a complete removable prosthesis that preferably, but not necessarily, is anchored on implants.

According to a further preferred embodiment of the invention the first 2 and the second 4 bite fork taper dorsally with regard to the backside of the mouth, following the natural convex shape curve of the tooth arcs 19, 20.

In a further preferred embodiment the instrument 1 is provided with a sliding part 14 over the extensions 5, 11, not only to keep the extensions of the first 2 and the second 4 bite fork together, but also as means for the operator of the instrument 1 to make the desired markings 28, 29, 30 on the extension 5, 11, corresponding to a certain number of positions of the lower jaw, i.e. at least the maximum protrusion 28 and retrusion 29, and the selected protrusion position 30 for the production of an oral correction device.

According to a further preferred embodiment of the invention the individual bite forks 2, 4 with extensions 5, 11, after removal from the mouth of the patient, can be placed back at later times if necessary or desired, in an accurate position in the mouth of the same patient to enable monitoring of individual patients over time.

In a further preferred embodiment, the components 2, 3, 4, 5, 11, 14, 18 thereof are made of materials suitable for automated production methods such as injection moulding of polymers, numerically controlled milling machines for metals, or robotics or other automated production methods, including those for sterilisable materials.

The invention relates to an instrument 1 for measuring the position of the lower jaw comprising a first 2 and a second 4 bite fork that are individually adaptable to the upper 19 and lower 20 tooth arch of a patient, whereby the first 2 and the second 4 bite fork are each attached to an excentric located extension 5, 11 that extends in a horizontal plane, so that the extensions 5, 11 extend outside the mouth of the patient in front of the anterior teeth when the first 2 and the second 4 bite fork are adapted to the upper 19 and lower 20 tooth arch, and whereby the extensions enable the bite forks 2, 4 to move with respect to one another in a horizontal movement direction 34, while the instrument 1 is placed in the patient's mouth and enables the relative position of the bite forks and thus the relative position of the upper and lower tooth arch 19, 20 to be registered outside the patient's mouth. The invention proposes that the first 2 and the second 4 bite fork have the same symmetric shape with regard to a midline position 32 of the first 2 and the second 4 bite fork, and wherein the extensions 5,11 are arranged eccentrically to the midline position 32 of the first 2 and the second 4 bite fork.

### Reference signs

- 1: Instrument
- 2: First bite fork
- 3: Retention holes
- 4: Second bite fork
- 5: First extension
- 6: Centreline
- 7: First notch
- 8: First egde
- 9: Metric scale
- 10: Longitudinal axis
- 11: Second extension
- 12: Second notch
- 13: Second edge
- 14: Sliding part
- 15: Window
- 16: Cutaway
- 17: Screw bolt
- 18: Set screw
- 19: Upper tooth arch
- 20: Lower tooth arch
- 21: Upper frontal incisor
- 22: Upper frontal incisor
- 23: Inter-incisor line, 23a Upper inter-incisor line, 23b Lower inter-incisor line
- 24: Lower frontal incisor
- 25: Lower frontal incisor
- 26: First thermoplastic layer
- 27: Second thermoplastic layer
- 28: Marking for maximum protrusion
- 29: Marking for maximum retrusion
- 30: Marking for the preferred position
- 31: Marking area
- 32: Midline position
- 33: Protrusion adjustment screw
- 34: Horizontal movement direction
- 35: Second screw bolt
- 36: End part
- 37: Seal

## Claims

1. Instrument (1) for measuring the position of the lower jaw, comprising a first (2) and a second (4) bite fork that are individually adaptable to the upper (19) and lower (20) tooth arch of a patient, whereby the first (2) and the second (4) bite fork are each attached to an extension (5, 11) that extends in a horizontal plane, so that the extensions (5, 11) extend outside the mouth of the patient in front of the anterior teeth when the first (2) and the second (4) bite fork are adapted to the upper (19) and lower (20) tooth arch, and whereby the extensions enable the bite forks (2, 4) to move with respect to one another in a horizontal movement direction (34) while the instrument (1) is placed in the patient's mouth and enables the relative position of the bite forks and thus the relative position of the upper and lower tooth arch (19, 20) to be registered outside the patient's mouth,
**characterised in that**
the first (2) and the second (4) bite fork have the same symmetric shape with regard to a midline position (32) of the first (2) and the second (4) bite fork, wherein the extensions (5, 11) are arranged eccentrically to the midline position (32) of the first (2) and the second (4) bite fork.

2. Instrument (1) for measuring the position of the lower jaw according to claim 1, **characterised in that** a first thermoplastic layer (26) is releasably attachable on the first bite fork (2) and a second thermoplastic layer (27) is releasably attachable on the second bite fork (4).

3. Instrument (1) for measuring the position of the lower jaw according to claim 1, **characterised in that** a first thermoplastic layer (26) is arranged on the first bite fork (2) and a second thermoplastic layer (27) is arranged on the second bite fork (4).

4. Instrument (1) for measuring the position of the lower jaw according to claim 2 or 3, **characterised in that** the thermoplastic layers (26, 27) can be made malleable by warming up.

5. Instrument (1) for measuring the position of the lower jaw according to one of the claims 2 to 4, **characterised in that** the first (2) and the second (4) bite fork have projections or retention holes (3) to fix the thermoplastic layers (26, 27) or any appropriate curing moulding compound on the first (2) and the second (4) bite fork.

6. Instrument (1) for measuring the position of the lower jaw according to one of the claims 1 to 5, **characterised in that** a protrusion adjustment screw (33) is arranged between the extensions (5, 11) of the first (2) and the second (4) bite fork controlling the movement of the extensions (5, 11) with respect to one another in the horizontal movement direction (34).

7. Instrument (1) for measuring the position of the lower jaw according to claim 6, **characterised in that** the protrusion adjustment screw (33) is arranged at the end of the extensions (5, 11) of the first (2) and the second (4) bite fork with a rotational axis in the horizontal direction of the movement (34).

8. Instrument (1) for measuring the position of the lower jaw according to one of the claims 1 to 7, **characterised in that** a set screw (18) fixes the first (2) and second (4) bite fork so that the first (2) and the second (4) bite fork do not slide freely with respect to one another in the horizontal movement direction (34).

9. Instrument (1) for measuring the position of the lower jaw according to claim 8, **characterised in that** the set screw (18) is arranged on the right side or left side of the extensions (5, 11) with a rotational axis in the horizontal plane locking the movement of the extensions (5, 11) with respect to one another.

10. Instrument (1) for measuring the position of the lower jaw according to one of the claims 1 to 9, **characterised in that** the vertical height of the first (2) and the second (4) bite fork with regard to the horizontal plane can be increased by click-on steps.

11. Instrument (1) for measuring the position of the lower jaw according to one of the claims 1 to 10, **characterised in that** the extensions (5, 11) of the first (2) and the second (4) bite fork are detachable so that the extensions (5, 11) can be attached to another third (2a) and fourth bottom (4a) bite fork.

12. Method for the use of an instrument (1) according to one of the previous claims, comprising at least the following steps:
1) Warming up a first (26) and a second (27) thermoplastic layer to make them malleable;
2) Introduction of the first bite fork (2) against the upper tooth arch (19) by putting the first thermoplastic layer (26) or curing moulding compound on the upper tooth arch (19) of the patient whereby the inter-incisor line (23a) between the two upper central frontal incisors (21, 22) is in line with a midline position (32) of the first (2) and the second (4) bite fork and whereby the outermost vestibular plane of these upper frontal incisors (21, 22) is positioned against a first edge (8) with a first notch (7), again with the inter-incisor line (23a) between both frontal incisors (21, 22) in line with the first notch (7);
3) Introduction of the second bite fork (4) against the lower tooth arch (20) by putting the second thermoplastic layer (27) or curing moulding compound on the lower tooth arch (20) of the patient whereby the inter-incisor line (23b) between the two lower central frontal incisors (24, 25) is in line with a midline position (32) of the first (2) and the second (4) bite fork and whereby the outermost vestibular plane of these lower frontal incisors (24, 25) is positioned against a second edge (13) with a second notch (12), again with the inter-incisor line (23b) between both frontal incisors (24, 25) in line with the second notch (12);;
4) Letting the thermoplastic layers (26, 27) or curing moulding compound harden until the upper (21, 22) and lower (24, 25) frontal incisors are positioned on the first (2) and the second (4) bite fork respectively;
5) Loosening of the set screw (18) that fixes the first (2) and the second (4) bite fork so that the first (2) and the second (4) bite fork can slide with respect to one another in a horizontal movement direction (34), whereby the movement of the extensions (5, 11) with respect to one another in the horizontal movement direction (34) is controlled with the protrusion adjustment screw (33);
6) Gradual and gentle forward movement of the patient's lower jaw from a backward position to a preferred position (30) in order to then register this position (30) for the patient between the front and back position on the instrument (1) by marking ;
7) Screwing in the set screw (18) on the instrument (1) so that the bite forks (2, 4) no longer move with respect to one another;
8) Taking the instrument (1) out of the mouth and using the instrument as an aid for the production of an oral correction device for the patient;

13. Method for the production of an instrument (1) according to any one of claims 1 to 11, comprising the following steps:
1) Provision of a first (2) and a second (4) bite fork;
2) Inserting the first (2) and the second (4) bite fork to a mould and closing the mould;
3) Injection of an overmould in the mould to produce a thermoplastic layer (26, 27) on the first (2) and the second (4) bite fork;
4) Setting of the overmould in the mould;
5) Ejection of the first (2) and the second (4) bite fork from the mould.

14. Method for the production of an instrument (1) for measuring the position of the lower jaw according to claim 13, **characterised in that** the first (2) and the second (4) bite fork are preheated before the injection of the overmould in the mould.

## Patentansprüche

1. Instrument (1) zum Messen der Position des Unterkiefers, aufweisend eine erste (2) und eine zweite (4) Bissgabel, die einzeln an den oberen (19) und unteren (20) Zahnbogen eines Patienten anpassbar sind, wobei die erste (2) und die zweite (4) Bissgabel jeweils an einen Fortsatz (5, 11) angebracht sind, der sich in einer Horizontalebene erstreckt, so dass die Fortsätze (5, 11) außerhalb des Munds des Patienten vor den Vorderzähnen verlaufen, wenn die erste (2) und die zweite (4) Bissgabel an den oberen (19) und den unteren (20) Zahnbogen angepasst sind, und wobei die Fortsätze es den Bissgabeln (2, 4) ermöglichen, sich in Bezug zueinander in einer horizontalen Bewegungsrichtung (34) zu bewegen, während das Instrument (1) im Mund des Patienten platziert wird, und es der Relativposition der Bissgabeln und somit der Relativposition des oberen und des unteren Zahnbogens (19, 20) ermöglichen, außerhalb des Munds des Patienten registriert zu werden,
**dadurch gekennzeichnet, dass**
die erste (2) und die zweite (4) Bissgabel die gleiche symmetrische Form hinsichtlich einer Mittellinienposition (32) der ersten (2) und der zweiten (4) Bissgabel haben, wobei die Fortsätze (5, 11) außermittig zu der Mittellinienposition (32) der ersten (2) und der zweiten (4) Bissgabel angeordnet sind.

2. Instrument (1) zum Messen der Position des Unterkiefers nach Anspruch 1, **dadurch gekennzeichnet, dass** eine erste thermoplastische Schicht (26) lösbar an der ersten Bissgabel (2) anbringbar ist, und eine zweite thermoplastische Schicht (27) lösbar an der zweiten Bissgabel (4) anbringbar ist.

3. Instrument (1) zum Messen der Position des Unterkiefers nach Anspruch 1, **dadurch gekennzeichnet, dass** eine erste thermoplastische Schicht (26) an der ersten Bissgabel (2) angeordnet ist und eine zweite thermoplastische Schicht (27) an der zweiten Bissgabel (4) angeordnet ist.

4. Instrument (1) zum Messen der Position des Unterkiefers nach Anspruch 2 oder 3, **dadurch gekennzeichnet, dass** die thermoplastischen Schichten (26, 27) durch Erwärmen formbar gemacht werden können.

5. Instrument (1) zum Messen der Position des Unterkiefers nach einem der Ansprüche 2 bis 4, **dadurch gekennzeichnet, dass** die erste (2) und die zweite (4) Bissgabel Vorsprünge oder Haltelöcher (3) aufweisen, um die thermoplastischen Schichten (26, 27) oder eine beliebige, geeignete aushärtende Formverbindung an der ersten (2) und der zweiten Bissgabel (4) zu befestigen.

6. Instrument (1) zum Messen der Position des Unterkiefers nach einem der Ansprüche 1 bis 5, **dadurch gekennzeichnet, dass** eine Vorsprungsstellschraube (33) zwischen den Fortsätzen (5, 11) der ersten (2) und der zweiten (4) Bissgabel angeordnet ist, welche die Bewegung der Fortsätze (5, 11) in Bezug aufeinander in der horizontalen Bewegungsrichtung steuert.

7. Instrument (1) zum Messen der Position des Unterkiefers nach Anspruch 6, **dadurch gekennzeichnet, dass** die Vorsprungsstellschraube (33) am Ende der Fortsätze (5, 11) der ersten (2) und zweiten (4) Bissgabel mit einer Drehachse in Horizontalrichtung der Bewegung (34) angeordnet ist.

8. Instrument (1) zum Messen der Position des Unterkiefers nach einem der Ansprüche 1 bis 7, **dadurch gekennzeichnet, dass** eine Feststellschraube (18) die erste (2) und die zweite (4) Bissgabel derart fixiert, dass die erste (2) und die zweite (4) Bissgabel in der horizontalen Bewegungsrichtung (34) in Bezug aufeinander nicht ungehindert gleiten.

9. Instrument (1) zum Messen der Position des Unterkiefers nach Anspruch 8, **dadurch gekennzeichnet, dass** die Feststellschraube (18) auf der rechten Seite oder der linken Seite der Fortsätze (5, 11) angeordnet ist, wobei eine Drehachse in der Horizontalebene die Bewegung der Fortsätze (5, 11) in Bezug zueinander sperrt.

10. Instrument (1) zum Messen der Position des Unterkiefers nach einem der Ansprüche 1 bis 9, **dadurch gekennzeichnet, dass** die Vertikalhöhe der ersten (2) und der zweiten (4) Bissgabel hinsichtlich der Horizontalebene durch "Click-on"-Schritte vergrößert werden kann.

11. Instrument (11) zum Messen der Position des Unterkiefers nach einem der Ansprüche 1 bis 10, **dadurch gekennzeichnet, dass** die Fortsätze (5, 11) der ersten (2) und der zweiten (4) Bissgabel lösbar sind, so dass die Fortsätze (5, 11) an einer weiteren dritten (2a) und vierten (4a) Bissgabel angebracht werden können.

12. Verfahren zur Verwendung eines Instruments (1) nach einem der vorstehenden Ansprüche, umfassend zumindest die folgenden Schritte:
1) Aufwärmen einer ersten (26) und einer zweiten (27) thermoplastischen Schicht, um sie formbar zu machen;
2) Einbringen der ersten Bissgabel (2) gegen den oberen Zahnbogen (19) durch Auftragen der ersten thermoplastischen Schicht (26) oder der härtenden Formverbindung auf den oberen Zahnbogen (19) des Patienten, wodurch die innere Schneidezahnlinie (23a) zwischen den zwei oberen Frontschneidezähnen (21, 22) mit einer Mittellinienposition (32) der ersten (2) und der zweiten (4) Bissgabel fluchtet, und wodurch die äußerste Vestibularebene dieser oberen Frontschneidezähne (21, 22) gegen eine erste Kante (8) mit einer ersten Kerbe (7) positioniert wird, wobei wiederum die innere Schneidezahnlinie (23a) zwischen beiden Frontschneidezähnen (21, 22) mit der ersten Kerbe (7) fluchtet;
3) Einbringen der zweiten Bissgabel (4) gegen den unteren Zahnbogen (20) durch Auftragen der zweiten thermoplastischen Schicht (27) oder der härtenden Formverbindung auf den unteren Zahnbogen (20) des Patienten, wodurch die innere Schneidezahnlinie (23b) zwischen den zwei unteren Frontschneidezähnen (24, 25) mit einer Mittellinienposition (32) der ersten (2) und der zweiten (4) Bissgabel fluchtet, und wodurch die äußerste Vestibularebene dieser unteren Frontschneidezähne (24, 25) gegen eine zweite Kante (13) mit einer zweiten Kerbe (12) positioniert wird, wobei die innere Schneidezahnlinie (23b) wiederum zwischen beiden Frontschneidezähnen (24, 25) mit der zweiten Kerbe (12) fluchtet;
4) Aushärten-Lassen der thermoplastischen Schichten (26, 27) oder der härtenden Formverbindung, bis die oberen (21, 22) und die unteren (24, 25) Frontschneidezähne auf der ersten (2) bzw. der zweiten (4) Bissgabel positioniert sind;
5) Lockern der Feststellschraube (18), die die erste (2) und die zweite Bissgabel (4) fixiert, so dass die erste (2) und die zweite (4) Bissgabel in einer horizontalen Bewegungsrichtung (34) in Bezug aufeinander gleiten können, wodurch die Bewegung der Fortsätze (5, 11) in Bezug aufeinander in der horizontalen Bewegungsrichtung (34) mit der Vorsprungsstellschraube (33) gesteuert wird;
6) Schrittweises und behutsames Vorwärtsbewegen des Unterkiefers des Patienten aus einer hinteren Position in eine bevorzugte Position (30), um dann diese Position (30) für den Patienten zwischen der vorderen und hinteren Position auf dem Instrument (1) durch Markieren zu registrieren;
7) Einschrauben der Feststellschraube (18) an das Instrument (1), so dass sich die Bissgabeln (2, 4) in Bezug aufeinander nicht mehr bewegen;
8) Entnehmen des Instruments (1) aus dem Mund und Verwenden des Instruments als Hilfe zur Herstellung einer oralen Korrekturvorrichtung für den Patienten.

13. Verfahren zur Herstellung eines Instruments (1) nach einem der Ansprüche 1 bis 11, umfassend die folgenden Schritte:
1) Bereitstellen einer ersten (2) und zweiten (4) Bissgabel;
2) Einlegen der ersten (2) und der zweiten (4) Bissgabel in ein Formwerkzeug und Schließen des Formwerkzeugs;
3) Einspritzen einer Umspritzung in das Formwerkzeug, um eine thermoplastische Schicht (26, 27) auf der ersten (2) und der zweiten (4) Bissgabel herzustellen;
4) Einsetzen der Umspritzung in das Formwerkzeug;
5) Ausstoßen der ersten (2) und der zweiten (4) Bissgabel aus dem Formwerkzeug.

14. Verfahren zur Herstellung eines Instruments (1) zum Messen der Position des Unterkiefers nach Anspruch 13, **dadurch gekennzeichnet, dass** die erste (2) und die zweite (4) Bissgabel vor dem Einspritzen der Umspritzung in dem Formwerkzeug vorerwärmt werden.

## Revendications

1. Instrument (1) permettant de mesurer la position de la mâchoire inférieure, l'instrument comprenant un premier (2) et un deuxième (4) porte-empreinte pouvant être adaptés individuellement aux arcades dentaires supérieure (19) et inférieure (20) d'un patient, moyennant quoi le premier (2) et le deuxième (4) porte-empreinte sont chacun fixé à une extension (5, 11) s'étendant dans un plan horizontal, de sorte que les extensions (5, 11) s'étendent à l'extérieur de la bouche du patient devant les dents antérieures lorsque le premier (2) et le deuxième (4) porte-empreinte sont adaptés aux arcades dentaires supérieure (19) et inférieure (20), et moyennant quoi les extensions permettent aux porte-empreintes (2, 4) de se déplacer l'un par rapport à l'autre dans une direction horizontale de déplacement (34) pendant que l'instrument (1) est placé dans la bouche du patient et permet à la position relative des porte-empreintes, et ainsi à la position relative des arcades dentaires supérieure et inférieure (19, 20), d'être enregistrées à l'extérieur de la bouche du patient,
**caractérisé en ce que**
le premier (2) et le deuxième (4) porte-empreinte prennent la même forme symétrique par rapport à une position de ligne médiane (32) du premier (2) et du deuxième (4) porte-empreinte, les extensions (5, 11) étant agencées de façon excentrique par rapport à la position de ligne médiane (32) du premier (2) et du deuxième (4) porte-empreinte.

2. L'instrument (1) permettant de mesurer la position de la mâchoire inférieure selon la revendication 1, **caractérisé en ce qu'**une première couche thermoplastique (26) peut être fixée de manière détachable sur le premier porte-empreinte (2), et **en ce qu'**une seconde couche thermoplastique (27) peut être fixée de manière détachable sur le deuxième porte-empreinte (4).

3. L'instrument (1) permettant de mesurer la position de la mâchoire inférieure selon la revendication 1, **caractérisé en ce qu'**une première couche thermoplastique (26) est agencée sur le premier porte-empreinte (2), et **en ce qu'**une seconde couche thermoplastique (27) est agencée sur le deuxième porte-empreinte (4).

4. L'instrument (1) permettant de mesurer la position de la mâchoire inférieure selon les revendications 2 ou 3, **caractérisé en ce que** les couches thermoplastiques (26, 27) peuvent être rendues malléables au moyen d'un préchauffage.

5. L'instrument (1) permettant de mesurer la position de la mâchoire inférieure selon l'une des revendications 2 à 4, **caractérisé en ce que** le premier (2) et le deuxième (4) porte-empreinte comportent des saillies ou des trous de retenue (3) destinés à fixer les couches thermoplastiques (26, 27), ou tout autre composé de moulage par durcissement approprié, sur le premier (2) et le deuxième (4) porte-empreinte.

6. L'instrument (1) permettant de mesurer la position de la mâchoire inférieure selon l'une des revendications 1 à 5, **caractérisé en ce qu'**une vis (33) de réglage de saillie est agencée entre les extensions (5, 11) du premier (2) et du deuxième (4) porte-empreinte, afin de réguler le déplacement des extensions (5, 11) l'une par rapport à l'autre dans la direction horizontale de déplacement (34).

7. L'instrument (1) permettant de mesurer la position de la mâchoire inférieure selon la revendication 6, **caractérisé en ce que** la vis (33) de réglage de saillie est agencée au niveau de l'extrémité des extensions (5, 11) du premier (2) et du deuxième (4) porte-empreinte, et comporte un axe de rotation dans la direction horizontale de déplacement (34).

8. L'instrument (1) permettant de mesurer la position de la mâchoire inférieure selon l'une des revendications 1 à 7, **caractérisé en ce que** une vis de pression (18) fixe le premier (2) et le deuxième (4) porte-empreinte, de sorte que le premier (2) et le deuxième (4) porte-empreinte ne coulissent pas librement l'un par rapport à l'autre dans la direction horizontale de déplacement (34).

9. L'instrument (1) permettant de mesurer la position de la mâchoire inférieure selon la revendication 8, **caractérisé en ce que** la vis de pression (18) est agencée du côté droit ou du côté gauche des extensions (5, 11) et comporte un axe de rotation dans le plan horizontal, permettant de bloquer le déplacement des extensions (5, 11) l'une par rapport à l'autre.

10. L'instrument (1) permettant de mesurer la position de la mâchoire inférieure selon l'une des revendications 1 à 9, **caractérisé en ce que** la hauteur verticale du premier (2) et du deuxième (4) porte-empreinte, par rapport au plan horizontal, peut être augmentée à l'aide de plusieurs crans de cliquage.

11. L'instrument (1) permettant de mesurer la position de la mâchoire inférieure selon l'une des revendications 1 à 10, **caractérisé en ce que** les extensions (5, 11) du premier (2) et du deuxième (4) porte-empreinte sont détachables, de sorte que les extensions (5, 11) puissent être attachées à des troisième (2a) et quatrième (4a) porte-empreintes différents.

12. Procédé d'utilisation d'un instrument (1) selon l'une des revendications précédentes, le procédé comprenant au moins les étapes suivantes :
1) Préchauffer une première (26) et une seconde (27) couche thermoplastique, afin de les rendre malléables ;
2) Introduire le premier porte-empreinte (2) contre l'arcade dentaire supérieure (19) au moyen du placement de la première couche thermoplastique (26), ou du composé de moulage par durcissement, sur l'arcade dentaire supérieure (19) du patient, moyennant quoi la ligne entre incisives (23a), entre les deux incisives frontales centrales supérieures (21, 22), est en ligne avec une position de ligne médiane (32) du premier (2) et du deuxième (4) porte-empreinte, et moyennant quoi le plan vestibulaire le plus externe desdites incisives frontales supérieures (21, 22) est positionné contre un premier bord (8) doté d'une première entaille (7), la ligne entre incisives (23a) étant encore entre les deux incisives frontales (21, 22) en ligne avec la première entaille (7) ;
3) Introduire le deuxième porte-empreinte (2) contre l'arcade dentaire inférieure (20) au moyen du placement de la seconde couche thermoplastique (27), ou du composé de moulage par durcissement, sur l'arcade dentaire inférieure (20) du patient, moyennant quoi la ligne entre incisives (23b), entre les deux incisives frontales centrales inférieures (24, 25), est en ligne avec une position de ligne médiane (32) du premier (2) et du deuxième (4) porte-empreinte, et moyennant quoi le plan vestibulaire le plus externe desdites incisives frontales inférieures (24, 25) est positionné contre un second bord (13) doté d'une seconde entaille (12), la ligne entre incisives (23b) étant encore entre les deux incisives frontales (24, 25) en ligne avec la seconde entaille (12) ;
4) Laisser durcir les couches thermoplastiques (26, 27), ou le composé de moulage par durcissement, jusqu'à ce que les incisives frontales supérieures (21, 22) et inférieures (24, 25) soient positionnées sur le premier (2) et le deuxième (4) porte-empreinte, respectivement ;
5) Desserrer la vis à pression (18) fixant le premier (2) et le deuxième (4) porte-empreinte, de sorte que le premier (2) et le deuxième (4) porte-empreinte puissent coulisser l'un par rapport à l'autre dans une direction horizontale de déplacement (34), moyennant quoi le déplacement des extensions (5, 11) l'une par rapport à l'autre dans la direction horizontale de déplacement (34) est régulé à l'aide de la vis (33) de réglage de saillie ;
6) Déplacer vers l'avant, de manière progressive et douce, la mâchoire inférieure du patient d'une position vers l'arrière à une position préférée (30), afin d'enregistrer ensuite ladite position (30) pour le patient, entre les positions avant et arrière, sur l'instrument (1) au moyen d'un marquage ;
7) Visser la vis à pression (18) sur l'instrument (1), de sorte que les porte-empreintes (2, 4) ne puissent plus se déplacer l'un par rapport à l'autre ;
8) Sortir l'instrument (1) de la bouche et utiliser l'instrument en tant qu'aide pour la production d'un dispositif correcteur buccal pour le patient.

13. Procédé de production d'un instrument (1) selon l'une quelconque des revendications 1 à 11, le procédé comprenant les étapes suivantes :
1) Mettre à disposition un premier (2) et un deuxième (4) porte-empreinte ;
2) Introduire le premier (2) et le deuxième (4) porte-empreinte dans un moule, et fermer le moule ;
3) Injecter un surmoule dans le moule afin de produire une couche thermoplastique (26, 27) sur le premier (2) et sur le deuxième (4) porte-empreinte ;
4) Laisser durcir le surmoule dans le moule ;
5) Éjecter le premier (2) et le deuxième (4) porte-empreinte hors du moule.

14. Le procédé de production d'un instrument (1) permettant de mesurer la position de la mâchoire inférieure selon la revendication 13, **caractérisé en ce que** le premier (2) et le deuxième (4) porte-empreinte sont préchauffés avant l'injection du surmoule dans le moule.
